# EUROPEAN PATENT APPLICATION

(11) **EP 2 952 497 A1**
(43) Date of publication of application: **09.12.2015**
(21) Application number: 14170960.0
(22) Date of filing: 03.06.2014
(51) Int. Cl.: C07B 63/02, C12Q 1/25

(54) **Method for neutralizing detrimental effects of thiolbearing compounds on metal catalysts**

(71) Applicant: Universität Basel, 4003 Basel (CH)
(72) Inventor: Ward, Thomas, 2072 St-Blaise (CH); Duerrenberger, Marc, 4419 Lupsigen (CH); Wilson, Yvonne Martha, 4056 Basel (CH); Pereira Nogueria, Elisa Sofia, 4750-798 Vila Cova BCL (PT)
(74) Representative: Schulz Junghans Patentanwälte PartGmbB

(57) **Abstract**

The present invention relates to the use of a thiol-neutralising agent for maintaining the catalytic activity of a metal catalyst in a cellular environment, wherein said cellular environment is characterized by the presence of a compound bearing a thiol group prior to addition of said thiol-neutralising agent.

## Description

The present invention relates to a process employing a thiol-neutralising agent to maintain the catalytic activity of a metal catalyst in cellular environment.

In many respects, organometallic- and enzymatic catalysis can be regarded as complementary. Over the past four decades, these two disciplines have by-and-large evolved independently. In recent years however, there has been an increasing interest in exploiting organometallic catalysis in the context of chemical biology, with the ultimate goal of integrating organometallic catalysts within the context of synthetic biology. To achieve this ambitious goal, however, the compatibility of organometallic catalysts within the sea of functionality present in a cell must be ensured.

In recent years, there have been a handful of reports claiming organometallic catalysis within living cells or in the presence of cell lysates. The catalytic efficiency of these processes remains modest at best, indicated by the very high catalyst loading used.

In this context, two potential challenges have been identified that may limit the versatility of organometallic catalysis in a cellular environment: i) the organometallic catalyst and the enzymes present in a cell suffer from mutual inhibition; ii) the glutathione (GSH hereafter), present in millimolar concentration in cells, poisons the soft transition metals widely used as organometallic catalysts.

The potential of artificial metalloenzymes has been investigated, to overcome the mutual inhibition. These hybrid catalysts result from incorporation of an organometallic moiety within a protein environment, thus conferring a well-defined second coordination sphere, reminiscent of natural enzymes.

The objective of the present invention is to provide means and methods that address *ex vivo* the GSH poisoning of organometallic catalysts, particularly precious metal containing catalysts.

This objective is attained by the subject-matter of the independent claims.

The present invention is based on the surprising finding that the negative or toxic effects of thiol-bearing compounds to the catalytic activity of metal catalysts can be reduced or prevented by the use of thiol-neutralising agents. Thiol-bearing compounds, particularly glutathione, are present in cellular environments such as cell lysates or cell free extracts. According to a first aspect of the invention, the use of a thiol-neutralising agent for maintaining the catalytic activity of a metal catalyst in a cellular environment is provided, wherein said cellular environment is characterized by the presence of a compound bearing a thiol group prior to addition of said thiol-neutralising agent.

The term "metal catalyst" refers to any catalyst that comprises a metal as catalytic entity, catalytic group or catalytic moiety.

In certain embodiments, the thiol-neutralising agent is selected from the group comprised of diamide (CAS number 10465-78-8), 3-phenyl-2-propynenitrile (CAS number 935-02-4), 2-bromoacetophenone (CAS number 70-11-1), phenylvinylsulfone (CAS number 5535-48-8) and a peroxysulfate.

An advantage of the thiol-neutralising agents, employed in the methods according to the invention, is that these agents are able to neutralise compounds bearing a thiol group, particularly glutathione, while on the other hand they do not substantially inhibit the catalytic activity of the metal catalyst.

In some embodiments, the cellular environment is selected from a cell, a cell compartment, a cell lysate and a cell free extract.

The term "cell lysate" in the context of the present specification, particularly refers to a fluid comprising the content of a lysed cell.

In some embodiments, the cellular environment is the soluble fraction of a cell lysate.

In some embodiments, the cell compartment is the cytosol of a cell.

In some embodiments, the cell is selected from the group comprised of a member of the genus *Escherichia* such as *E. coli,* a member of the genus *Saccharomyces* such as S. *cerevisiae,* a member of the genus *Schizosaccharomyces* such as *S*. *pombe,* a member of the genus *Pichia* such as *P*. *pastoris,* a member of the genus *Aspergillus* such as *A.niger,* a member of the genus *Bacillus* such as *B*. *subtilis* and a mammalian cell in cell culture such as a CHO (Chinese hamster ovary) cell or a HEK (Human Embryonic Kidney) 293 cell. In some embodiments, the cell compartment, the cell lysate or the cell free extract is derived from a cell as specified in this paragraph.

In certain embodiments, the metal catalyst comprises a transition metal atom. In certain embodiments, the transition metal atom is selected form rhodium, ruthenium, iridium, platinum, osmium and vanadium (Rh, Ru, Ir, Pt, Os and V).

In certain embodiments, the metal catalyst comprises an organic ligand. In some embodiments, the transition metal atom is attached or bound to the organic ligand. The nature of the bond between the metal atom and the organic ligand is covalent, ionic or coordinative in nature.

In certain embodiments, the organic ligand is selected from cyclopentadienyl, p-cymene and an organophosphine. In certain embodiments, the organic ligand is selected from an N-heterocyclic carbene, an amine, an amide, an arene (including cyclopentadienyl (Cp), pentamethylcyclopentadienyl (Cp*) and derivatives thereof), a pyridine, an alcohol and an arsine.

In some embodiments, the metal catalyst is a [Cp*Ir] complex.

In some embodiments, the metal catalyst is attached to a polypeptide, thereby forming an artificial metalloenzyme.

The term "artificial metalloenzyme" in the context of the present specification, particularly refers to a metal catalyst that is attached to, incorporated in, complexed by (coordinatively bound to) or embedded within a polypeptide.

In some embodiments, the metal catalyst is covalently or non-covalently attached or bound to the polypeptide. In some embodiments, the metal catalyst is specifically and non-covalently attached to the polypeptide with a dissociation constant of equal or less than 10⁻⁷ mol/l, 10⁻⁸ mol/l, 10⁻⁹ mol/l, 10⁻¹⁰ mol/l or 10⁻¹¹ mol/l.

In some embodiments, the polypeptide part of the artificial metalloenzyme is a protein known to incorporate an abiotic metal cofactor. In certain embodiments, the polypeptide is a variant or structural and/or functional homologue of carbonic anhydrase, albumin, papain, subtilisine, ribonuclease S or ferritin.

In some embodiments, the organic ligand comprises a biotin moiety.

In some embodiments, the polypeptide is a biotin binding protein. In some embodiments, the metal catalyst is non-covalently attached to biotin-binding protein via the biotin-moiety of the organic ligand, which is comprised within the metal catalyst.

A biotin binding protein in the context of the present specification refers to a polypeptide that is able to bind biotin or to bind a compound comprising a biotin moiety with a dissociation constant of equal or less than 10⁻⁷ mol/l, 10⁻⁸ mol/l, 10⁻⁹ mol/l, 10⁻¹⁰ mol/l or 10⁻¹¹ mol/l. Examples for such biotin-binding proteins include, without being restricted to, streptavidin (UniProt P22629), avidin (UniProt P02701), rhizavidin (UniProt Q8KKW2) or variants and functional homologues thereof.

UniProt numbers contained in the present specification refer to entries in the Protein knowledgebase (Swiss Institute of Bioinformatics).

In some embodiments, the biotin-binding protein is a molecule comprising or consisting of streptavidin, avidin, rhizavidin or a variant or functional homologue thereof.

In some embodiments, the metal catalyst is a biotinylated d⁶-Ir pianostool complex. In some embodiments, the metal catalyst is a [Cp*Ir(biot-*p*-L)Cl] complex. In some embodiments, the polypeptide consists of or comprises an amino acid sequence characterized by SEQ ID NO 1 (Streptavidin variant S112A) or SEQ ID NO 02 (streptavidin variant S112K).

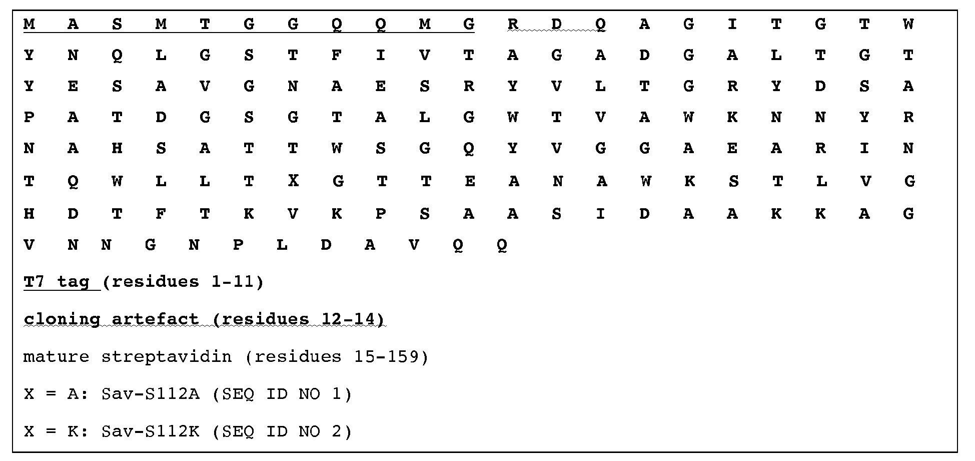

In some embodiments, a [Cp*Ir(biot-*p*-L)Cl] complex is attached via the biotin-moiety to streptavidin or a polypeptide consisting of or comprising an amino acid sequence characterized by SEQ ID NO 1 (Streptavidin variant S112A) or SEQ ID NO 02 (streptavidin variant S112K), thereby forming an artificial metalloenzyme. The formed artificial metalloenzyme is an artificial imine reductase, which is particularly able to catalyse the transfer hydrogenation of prochiral imines.

Where reference is made herein to a polypeptide characterized by a particular sequence, such reference is meant to also encompass polypeptides having a similar, or an identical, chemical functionality to the particular sequence, and showing a sequence identity of at least 70%, 80%, 90% or 95% to the referenced sequence.

In some embodiments, the compound bearing a thiol group is glutathione.

According to an alternative of this first aspect of the invention, a method for conducting a chemical reaction in a cellular environment, such as, by non-limiting example, a cell, a cell compartment, a cell lysate or a cell free extract is provided. The chemical reaction is catalysed by a metal catalyst, such as, by non-limiting example, a transition metal atom bearing metalloenzyme and the cellular environment comprises thiol compounds. The method includes adding a thiol-neutralising agent such as, by way of non-limiting example, diamide, 3-phenyl-2-propynenitrile, 2-bromoacetophenone phenylvinylsulfone or a peroxysulfate.

According to another aspect of the invention, a method for making a reaction mixture useful for being employed in a reaction is provided, wherein such reaction is catalyzed by a metal catalyst. The reaction takes place in a cellular environment and comprises the steps of:
- providing a cellular environment characterized by the presence of a compound bearing a thiol group, exemplified by, but not limited to, glutathione,
- adding to the reaction medium a thiol-neutralising agent as specified in the above aspect or embodiments of the invention, in an amount sufficient for substantially all of the compound bearing a thiol group to react with the thiol-neutralising agent, and
- adding a metal catalyst as specified in the above aspect or embodiments of the invention to the reaction medium.

According to yet another aspect of the invention, a reaction mixture is provided, particularly obtainable by a method according to the above aspect of the invention. The reaction mixture is comprised of a cell lysate or a cell free extract, a metal catalyst as specified in above aspects or embodiments of the invention, and a thiol-neutralising agent as specified in the above aspects or embodiments of the invention.

In certain embodiments, the reaction mixture is characterized by the presence of glutathione of not more than (≤) 5mmol/l, ≤ 2,5 mmol/l or ≤1 mmol/l. In certain embodiments, the glutathione concentration is ≤ 0,5 mmol/l, ≤0,25 mmol/l or ≤ 0,1 mmol/l.

According to a further aspect of the invention, a method for reacting an educt (reactant) to a product in a cellular environment is provided. The reaction is catalysed by a metal catalyst, and the method comprises the steps of:
- providing a reaction mixture as specified in the above aspect or embodiments of the invention,
- adding to the reaction mixture an educt (reactant) that is a substrate of the metal catalyst.

In some embodiments, the reaction is selected from hydrogenation, transfer hydrogenation, allylic alkylation, sulfoxidation and dihydroxylation.

In some embodiments, the educt is a prochiral substrate of the metal catalyst. In some embodiments, the prochiral substrate is converted by the metal catalyst into a chiral product. In some embodiments, the educt or substrate is 6,7-dimethoxy-1-methyl-3,4-dihydroisoquinoline. In some embodiments, the product is (1*S*)-6,7-dimethoxy-1-methyl-1,2,3,4-tetrahydroisoquinoline or (1*R*)-6,7-dimethoxy-1-methyl-1,2,3,4-tetrahydroisoquinoline. A prochiral substrate or compound is a compound having a sp3 hybridised double bond that can be converted to a chiral compound in a single reaction. Prochirality in the sense used in the context of the present specification is a quality attributable to a bond in a molecule. Thus, a prochiral bond may be comprised in a molecule that has a chiral centre in another part of the molecule.

According to another aspect of the invention, a method for determining the catalytic activity of a metal catalyst in a cellular environment is provided, wherein the method comprises the steps of:
- providing a reaction mixture as specified in the above aspects or embodiments of the invention, wherein said metal catalyst is attached to a polypeptide as specified in the above embodiments of the invention,
- adding to said reaction mixture an educt, wherein the educt is a substrate of the metal catalyst,
- determining the amount of the educt or the product, and
- optionally, comparing the amount to a standard.

In some embodiments, the educt is a prochiral substrate of the metal catalyst. In some embodiments, the prochiral substrate is converted by the metal catalyst into a chiral product. In some embodiments, the substrate is converted by the metal catalyst into the product in a reaction selected from hydrogenation, transfer hydrogenation, allylic alkylation, sulfoxidation and dihydroxylation.

In some embodiments, the polypeptide is expressed in the cellular environment before the metal catalyst is added to the cellular environment. In some embodiments, the polypeptide is expressed in a cell, particularly in the cytosol of the cell, and the cell is lysed prior to the method of the invention being applied, yielding a cell lysate of the cell comprising the polypeptide.

As an advantage of the above embodiment, the method of the invention according to this last mentioned aspect allows the screening of polypeptides or variants thereof for their suitability as polypeptide components for artificial metalloenzymes without need to purify the polypeptides or their variants from the cell lysate.

### Description of the figures

- Fig. 1: shows SDS PAGE of a cell free extract from an *E coli* culture expressing S112A Sav (right lane: pure S112A Sav sample) biotin-4-fluorescein staining to reveal biotin-binding proteins (left) and Coomassie-blue staining (right). Under the conditions used, tetrameric Sav is not denatured.
- Fig. 2: shows the thiol-neutralising agents tested for neutralising glutathione (top oxidizing agents, bottom Michael acceptors).

### Examples

The present invention enables transition metal catalysis in the presence of cellular debris, particularly *E. coli* cell free extracts and cell lysates. This is hampered by the presence of thiols, mainly present in form of glutathione (GSH), which poison precious metal catalysts. To overcome this, selection of oxidizing agents and electrophiles were evaluated towards their potential to neutralize the detrimental effect of GSH. While organometallic catalysts were severely inhibited by cellular debris, embedding the organometallic moiety within a host protein led to promising results in the presence of some neutralizing agents. The asymmetric imine reductase based on the incorporation of a biotinylated iridium pianostool complex within streptavidin isoforms was used as model reaction. Compared to purified protein samples, it could be shown that pretreatment of cell free extracts and cell lysates containing Sav-mutants with diamide affords up to > 100 TON's and only a modest erosion of enantioselectivity.

In order to identify the most suitable GSH neutralizing agent in the context of organometallic catalysis, the IrCp*-catalyzed asymmetric transfer hydrogenation was selected as a benchmark reaction. A versatile artificial transfer hydrogenase based on the incorporation of a biotinylated d6-Ir pianostool complex [Cp*Ir(biot-*p*-L)Cl] within streptavidin (Sav hereafter) was used for the transfer hydrogenation of prochiral imines. Genetic optimization revealed that mutation at position S112 offered an attractive means to improve (and invert) the enantioselectivity: [Cp*Ir(biot-*p*-L)Cl] c S112A Sav and [Cp*Ir(biot-*p*-L)Cl] c S112K Sav yield the opposite enantiomers of salsolidine in 96 and 78 % ee under optimized conditions using purified Sav.

For screening purposes, *E. coli* cell-free extracts and cell lysates were selected. Following over-expression of a Sav isoform in the *E. coli* strain BL21(DE3) pLysS, the cell pellet was lysed by freezing followed by addition of TRIS-HCl buffer containing DNAse I and PMSF (phenylmethylsulfonylfluoride) as a protease inhibitor. Catalysis was either performed in the resulting cell lysate or in cell free extract, which was obtained by centrifugation and lyophilisation of the supernatant to yield a brown powder. The dried cell free extract (cfe) was stored at 4 °C until use. The biotin-binding site concentration of the cell lysates and cfe was determined using the biotin-4-fluorescein assay. The total thiol concentration of the cfe was estimated to approximately 500 µM using Ellman's reagent. As Sav, which is devoid of cysteines, represents > 40% of the proteins present in the cfe, we conclude that most of the cysteines quantified by the Ellman test are contained in the glutathione present in cfe.

For comparison purposes, catalytic transfer hydrogenase experiments using [Cp*Ir(biot-*p-*L)CI] were carried out with i) purified Sav samples, ii) purified Sav samples spiked with GHS, iii) cfe containing Sav isoforms iv) cfe containing no Sav (resulting from an *E coli* culture using an plasmid devoid of the Sav gene) and cell lysates containing Sav isoforms.

Initial experiments were performed using 50 µmol/l [Cp*Ir(biot-*p*-L)Cl], 100 µmol/l biotin binding sites (i.e. 25 µmol/l tetrameric Sav), 1 M HCO₂Na in MOPS buffer (100 µmol/l, pH 7) (see scheme 1). For comparison purposes, the imine reduction was performed with [Cp*Ir(biot-*p*-L)Cl]; [Cp*Ir(biot-*p*-L)Cl] c S112A Sav and [Cp*Ir(biot-*p*-L)Cl] c S112K Sav, cf. scheme 1. The results are summarized in table 1.

Catalysis results from experiments performed at 250 µmol/l Sav and 50 µmol/l Sav are similar, suggesting that the ATHase is well suited to operate at low concentrations (table 1, compare entries 1-3 to 4-6). Upon addition of 2.5 mM GSH, all three catalytic systems were severely inhibited and showed no activity towards the reduction of the salsolidine precursor (1-methyl-6, 7-dimethoxy-3, 4-dihydroisoquinoline) present at 10 mM (Table 1, entries 7-9). In contrast, addition of GSSG had a less negative impact on the reaction, although in case of the bare Ir-complex and the S112K mutant the conversion was found to be modest (Table 1, entries 10-12). Without wishing to be bound by theory, the inventors speculate that oxidizing GSH or derivatizing its thiol function could -at least partially- prevent inhibition of the hybrid catalysts.

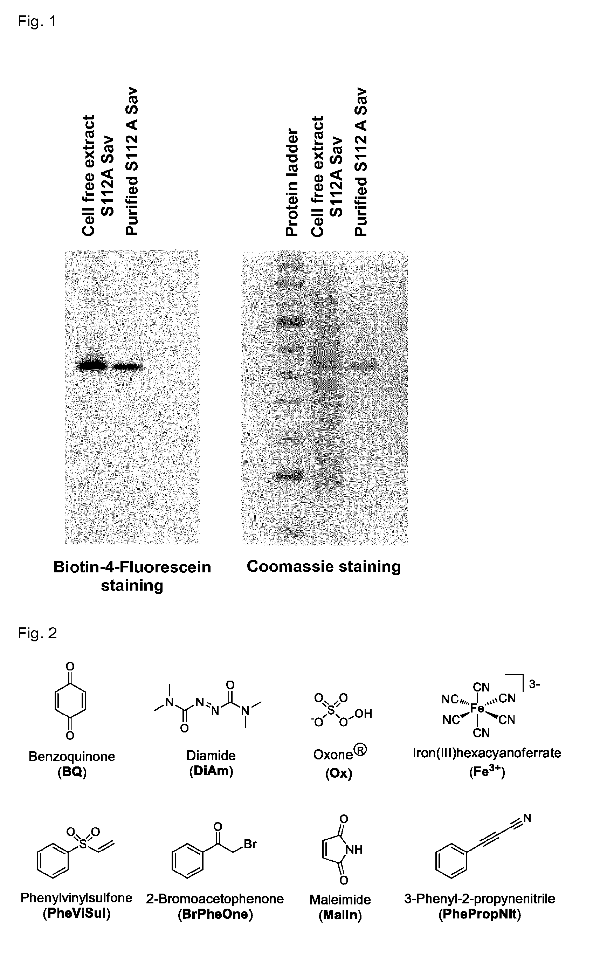

Michael acceptors and oxidizing agents known to react with GSH were selected to yield the corresponding thioether and disulfide, respectively. The following Michael acceptors were tested: maleinimide (Malln), 2-bromo-1-phenylethanone (BrPheOne), phenyl-vinylsulfone (PheViSul) and 3-Phenyl-2-propynenitrile (PhePropNit). The following oxidizing agents were selected: oxone (Ox), 1,4-benzoquinone (BQ), K₃[Fe(CN)₆] Fe³⁺, and diamide (DiAm) (figure 2). For this purpose, solutions either with or without a Sav isoform were spiked with 2.5 mmol/l GSH and incubated overnight in presence of different concentrations of a particular GSH neutralizing agent before adding the Ir-catalyst. The reactions were initiated by addition of the imine substrate and run for 2 days (see Table 2).

The main findings of this screening include:
1) Fe³⁺ and BQ are not compatible with any of the three catalytic systems: even in the absence of GSH, no or low conversion was observed.
2) Malln exhibits limited compatibility with the Ir-catalyst, especially when the latter is embedded within Sav.
3) PhePropNit is most effective when applied at a ratio PhePropNit/GSH 2:1. An equimolar amount has no benefit on the reaction whereas four equivalents led to a decrease in conversion and enantioselectivity in the case of the S112K mutant.
4) Despite the fact that H₂O₂ is commonly used to oxidize GSH, Ox was favoured for this purpose. Ox was found to be more compatible with the experimental set-up as the presence of catalases in the cfe lead to significant gushing upon addition of H₂O₂. Strikingly, although Ox is a two electron oxidant, its efficacy is most pronounced with four equivalents vs. GSH. This behavior may be traced back to the fact that, in presence of formate, GSH is oxidized to the corresponding sulfonic acid rather than the disulfide GSSG.
5) The most effective GSH neutralizing agents are BrPheOne, PheViSul and DiAm. Compared to reactions where GSH is absent, all these agents led to comparable conversions and enantioselectivities in most cases when incubated for either 2 or 15 hours to GSH-spiked solutions prior to catalysis. Increasing the concentration of the GSH neutralizing agents leads to less consistent results. The outcome of the reaction depends on the Sav-mutant (if any) is used. For example, DiAm is fully compatible with the artificial metalloenzymes, but not with free [Cp*Ir(biot-*p*-L)Cl] as increasing concentrations led to a decrease in conversion. A similar behaviour is observed with BrPheOne when no Sav or the S112K mutant was present. On the other hand, PheViSul does not affect the free Ir-complex, but limited compatibility was observed with the hybrid catalysts especially in absence of GSH.

With regard to these results, the following properties of the GSH neutralizing agents may have to be considered: i) efficiency of GSH neutralizing ii) (mutual) inhibition between the catalyst and the GSH neutralizing agent (compatibility), iii) inhibition of the catalyst and the product of the GSH neutralization (disulfide and thioether respectively), iv) reduction of GSSG to GSH by the Ir-catalyst and v) derivatization of Sav amino acid side chains by the Michael acceptors.

Next, the most promising glutathione neutralizing agents BrPheOne, PheViSul and DiAm were tested in presence of cell free extracts (table 2). While the bare catalyst was inactive, up to thirty turnovers were obtained for the [Cp*Ir(biot-*p*-L)Cl] c S112K Sav variant without pretreatment of the cfe with a GSH neutralizing agent. This clearly demonstrates the efficacy of the second coordination sphere to protect the precious metal from cell debris (table 2). In the presence of a GSH neutralizing agent, the catalytic performance could be improved significantly for the artificial metalloenzyme. DiAm proved most effective at concentrations of 5-10 mmol/l leading up to 126 turnovers in case of the S112A mutant and 136 turnovers with the S112K mutant (table 2, entry 14 and 15). This amounts to up to 97 % recovery of the original activity of these catalysts, without loss of selectivity. Reducing the pre-incubation time to 2 hours yielded nearly identical results (table 2, entry 16 and 17). On the other hand, none of the three GSH neutralizing agents had a beneficial effect on the bare Ir-complex in cfe's : conversions do not exceed 5 % (Table 2, entry 7, 10 and 13).

Recent saturation kinetic studies demonstrate that the [Cp*Ir(biot-*p*-L)Cl] c S112A Sav ATHase exhibits higher reaction rates than its [Cp*Ir(biot-*p*-L)Cl] c S112K Sav counterpart (*k*_{cat} = 11.4 min⁻¹ vs. *k*_{cat} = 2.6 min⁻¹). Although these kinetic profiles are reflected in the initial screening experiments in presence of purified Sav, the S112K variant generally yields better conversions in presence of pretreated cfe. Assuming that the neutralization of GSH in the cellular environment is as efficient as in the presence of purified protein, this finding suggests that GSH may be the main, but not the sole inhibitor of the precious metal catalyst. It thus appears that the presence of a cationic residue shields the metal better towards cellular debris detrimental to the precious metal cofactor.

The ATHase was tested with cell lysates. Again here, the best results were achieved with DiAm yielding up to 48 turnovers (S112A) and 84 (S112K) turnovers, respectively (table 2, entry 22 and 23). Compared to experiments performed with cfe's lower conversions were obtained and in the case of the S112A-ATHase also a slight decrease of enantioselectivity was observed. The S112K mutant shows no degradation in agreement with the assumption that the latter better protects the Ir-center from inhibitors.

In conclusion, a suitable procedure has been developed to enable catalysis in cfe's and to some extent in cell lysates. A proof-of-principle protocol could be established. It could be shown that diamide is a GSH oxidizing agent which was shown to be fully compatible with organometallic catalysis on cell free extracts as well as cell lysates. Importantly, it has been shown that diamide DiAm is compatible with living cells, including *E. coli,* causing neither lysis nor death. Its compatibility with a precious metal catalyst opens fascinating perspectives towards performing catalysis *in vivo* or on cell lysates.

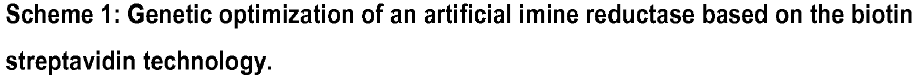

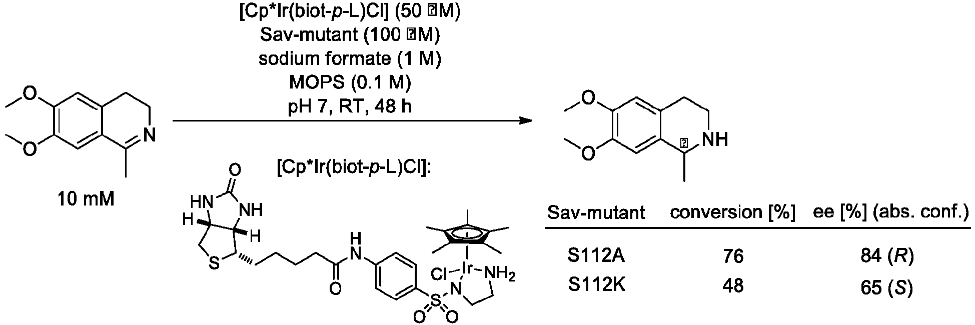

**Table 1. Catalysis with purified Sav at low catalyst concentration and in presence of GSH and GSSG at RT.**

| entry | Sav-mutant | [Sav] (mM) | [Ir] (mM) | [formate] (M) | [GSH] (mM) | [GSSG] (mM) | [substrate] (mM) | time (h) | conversion^{b} (%) | ee^{a,b} (%) |
|---|---|---|---|---|---|---|---|---|---|---|
| 1 | no | 125 | 250 | 3 | 0 | 0 | 50 | 4.5 | quant. | 0 |
| 2 | S112A | 125 | 250 | 3 | 0 | 0 | 50 | 4.5 | 89±1 | 90±1 |
| 3 | S112K | 125 | 250 | 3 | 0 | 0 | 50 | 4.5 | 70±3 | 75±1 |
| 4 | no | 25 | 50 | 1 | 0 | 0 | 10 | 48 | quant. | 0 |
| 5 | S112A | 25 | 50 | 1 | 0 | 0 | 10 | 48 | 69±5 | 81±1 |
| 6 | S112K | 25 | 50 | 1 | 0 | 0 | 10 | 48 | 48±1 | 62±2 |
| 7 | no | 25 | 50 | 1 | 2.5 | 0 | 10 | 48 | 0 | 0 |
| 8 | S112A | 25 | 50 | 1 | 2.5 | 0 | 10 | 48 | 0 | 0 |
| 9 | S112K | 25 | 50 | 1 | 2.5 | 0 | 10 | 48 | 0 | 0 |
| 10 | no | 25 | 50 | 1 | 0 | 2.5 | 10 | 48 | 19±1 | 0 |
| 11 | S112A | 25 | 50 | 1 | 0 | 2.5 | 10 | 48 | 50±1 | 84±2 |
| 12 | S112K | 25 | 50 | 1 | 0 | 2.5 | 10 | 48 | 15±1 | 47±3 |

| | | | | | | | | | | |
|---|---|---|---|---|---|---|---|---|---|---|
| a) positive ee values correspond to (*R*)-salsolidine and negative ee values correspond to (*S*)-salsolidine. b) ± values represent standard deviations resulting from triplicate measurements. Sav concentrations given relate to the concentration of the Sav tetramer. | | | | | | | | | | |

**Table 2. Selected results obtained from catalysis in presence of cell free extracts (cfe) and cell lysates. All reactions were performed with 50 mM [Cp*Ir(biot-p-L)Cl], 25 mM [Sav], 0.6 M MOPS pH 7 and 3 M sodium formate at RT for 48 h.**

| entry | Sav-mutant | origin | GSH neutralizing agent | Concentration (mM) | preincubation time (h) | conversion^{b} (%) | ee^{a, b} (%) |
|---|---|---|---|---|---|---|---|
| 1 | no | purified | no | - | - | 98±1 | 0 |
| 2 | S112A | purified | no | - | - | 95±3 | 82±2 |
| 3 | S112K | purified | no | - | - | 67±3 | -64±3 |
| 4 | no | cfe^{c} | no | - | - | 2±1 | ○ |
| 5 | S112A | cfe | no | - | - | 11±2 | 68±2 |
| 6 | S112K | cfe | no | - | - | 8±2 | -55±5 |
| 7 | no | cfe^{c} | BrAcPhe | 5 | 15 | 0 | - |
| 8 | S112A | cfe | BrAcPhe | 5 | 15 | 20±1 | 74±3 |
| 9 | S112K | cfe | BrAcPhe | 5 | 15 | 25±3 | -62±6 |
| 10 | no | cfe^{c} | PheViSul | 5 | 15 | 2±1 | 0 |
| 11 | S112A | cfe | PheViSul | 5 | 15 | 22±5 | 62±3 |
| 12 | S112K | cfe | PheViSul | 5 | 15 | 32±1 | -44±6 |
| 13 | no | cfe^{c} | DiAm | 10 | 15 | 4±1 | 0 |
| 14 | S112A | cfe | DiAm | 5 | 15 | 55±6 | 83±2 |
| 15 | S112K | cfe | DiAm | 5 | 15 | 48±5 | -60±4 |
| 16 | S112A | cfe | DiAm | 10 | 2 | 49±10 | 85±1 |
| 17 | S112K | cfe | DiAm | 10 | 2 | 42±1 | -64±6 |
| 18 | S112A | cell lysate | BrAcPhe | 10 | 15 | 6±1 | 59±17 |
| 19 | S112K | cell lysate | BrAcPhe | 10 | 15 | 31±12 | -66±1 |
| 20 | S112A | cell lysate | PheViSul | 10 | 15 | 9±1 | 54±11 |
| 21 | S112K | cell lysate | PheViSul | 10 | 15 | 20±5 | -42±6 |
| 22 | S112A | cell lysate | DiAm | 10 | 15 | 22±3 | 70±6 |
| 23 | S112K | cell lysate | DiAm | 10 | 15 | 43±7 | -68±1 |

| | | | | | | | |
|---|---|---|---|---|---|---|---|
| a) positive ee values correspond to (*R*)-salsolidine and negative ee values correspond to (*S*)-salsolidine, b) ± values represent standard deviations resulting from triplicate measurements c) cfe containing no Sav resulting from an *E coli* culture using an plasmid devoid of the Sav gene. | | | | | | | |

## Claims

1. Use of a thiol-neutralising agent selected from the group comprised of diamide, 3-phenyl-2-propynenitrile, 2-bromoacetophenone, phenylvinylsulfone and a peroxysulfate for maintaining the catalytic activity of a metal catalyst in a cellular environment, wherein said cellular environment is **characterized by** the presence of a compound bearing a thiol group prior to addition of said thiol-neutralising agent.

2. The use of a thiol-neutralising agent according to claim 1, wherein said cellular environment is selected from a cell, a cell compartment, a cell lysate and a cell free extract.

3. The use of a thiol-neutralising agent according to any one of the preceding claims, wherein said metal catalyst comprises a transition metal atom selected from Rh, Ru, Ir, Pt, Os and V.

4. The use of a thiol-neutralising agent according to any one of the preceding claims, wherein said metal catalyst comprises an organic ligand, particularly selected from cyclopentadienyl, petamethylcyclopentadienyl, p-cymene, phosphine, an organophosphine, an N-heterocyclic carbene, an amine, an amide, an arene (including Cp, Cp* and derivatives thereof), a pyridine, an alcohol and an arsine.

5. The use of a thiol-neutralising agent according to any one of the preceding claims, wherein said metal catalyst is attached to, incorporated in, complexed by (coordinatively bound to) or embedded within a polypeptide, thereby forming an artificial metalloenzyme.

6. The use of a thiol-neutralising agent according to claim 5, wherein said organic ligand comprises a biotin moiety, and said polypeptide is a biotin-binding protein, particularly streptavidin or variant thereof.

7. The use of a thiol-neutralising agent according to any one of the preceding claims, wherein said compound bearing a thiol group is glutathione.

8. A method for making a reaction mixture for a reaction catalysed by a metal catalyst in a cellular environment, said method comprising the steps of:
- providing a cellular environment as specified in claim 1 or 2 as a reaction medium,
- adding to said reaction medium a thiol-neutralising agent as specified in claim 1 in an amount sufficient to react substantially all of said compound bearing a thiol group with said thiol-neutralising agent, and
- adding a metal catalyst as specified in claim 1, 3, 4, 5, or 6 to said reaction medium.

9. A reaction mixture comprising a cell lysate or a cell free extract, a metal catalyst as specified in claim 1, 3, 4, 5, or 6, and a thiol-neutralising agent as specified in claim 1.

10. A method for a reaction of an educt to a product in a cellular environment, said reaction being catalysed by a metal catalyst, and said method comprising the steps of:
- providing a reaction mixture as specified in claim 8 or 9,
- adding to said reaction mixture an educt, wherein said educt is a substrate of said metal catalyst.

11. The method according to claim 10, wherein said reaction is selected from hydrogenation, transfer hydrogenation, allylic alkylation, sulfoxidation and dihydroxylation.

12. The method according to claim 10 or 11, wherein said educt is a prochiral substrate, and wherein said prochiral substrate is converted by said metal catalyst into a chiral product.

13. A method for determining the catalytic activity of a metal catalyst in a cellular environment, the method comprising the steps of:
- providing a reaction mixture as obtainable by a method according to claim 8 or 9, wherein said metal catalyst is attached to a polypeptide as specified in claim 5 or 6,
- adding to said reaction mixture an educt, wherein the educt is a substrate of said metal catalyst, said educt is converted by said metal catalyst into a product, for example in a reaction selected from hydrogenation, transfer hydrogenation, allylic alkylation, sulfoxidation and dihydroxylation, C-H activation or olefin metathesis,
- determining the amount of said educt or said product after a determined amount of time in which said conversion of educt to product is allowed to take place, and
- optionally, comparing said amount to a standard.

14. The method according to claim 13, wherein said educt is a prochiral substrate, and said product is a chiral molecule.

15. The method according to claim 13, wherein said polypeptide is expressed in said cellular environment before adding said metal catalyst.


SEQUENCE LISTING

<110>  Universitaet Basel

<120>  Method for neutralizing detrimental effects of thiol-bearing
       compounds on metal catalysts

<130>  uz197ep-1

<160>  2

<170>  PatentIn version 3.5

<210>  1
<211>  159
<212>  PRT
<213>  artificial

<220>
<223>  streptavidin-based organometallo enzyme

<400>  1

Met Ala Ser Met Thr Gly Gly Gln Gln Met Gly Arg Asp Gln Ala Gly
1               5                   10                  15

Ile Thr Gly Thr Trp Tyr Asn Gln Leu Gly Ser Thr Phe Ile Val Thr
            20                  25                  30

Ala Gly Ala Asp Gly Ala Leu Thr Gly Thr Tyr Glu Ser Ala Val Gly
        35                  40                  45

Asn Ala Glu Ser Arg Tyr Val Leu Thr Gly Arg Tyr Asp Ser Ala Pro
    50                  55                  60

Ala Thr Asp Gly Ser Gly Thr Ala Leu Gly Trp Thr Val Ala Trp Lys
65                  70                  75                  80

Asn Asn Tyr Arg Asn Ala His Ser Ala Thr Thr Trp Ser Gly Gln Tyr
                85                  90                  95

Val Gly Gly Ala Glu Ala Arg Ile Asn Thr Gln Trp Leu Leu Thr Ala
            100                 105                 110

Gly Thr Thr Glu Ala Asn Ala Trp Lys Ser Thr Leu Val Gly His Asp
        115                 120                 125

Thr Phe Thr Lys Val Lys Pro Ser Ala Ala Ser Ile Asp Ala Ala Lys
    130                 135                 140

Lys Ala Gly Val Asn Asn Gly Asn Pro Leu Asp Ala Val Gln Gln
145                 150                 155

<210>  2
<211>  159
<212>  PRT
<213>  artificial

<220>
<223>  streptavidin based metalloenzyme

<400>  2

Met Ala Ser Met Thr Gly Gly Gln Gln Met Gly Arg Asp Gln Ala Gly
1               5                   10                  15

Ile Thr Gly Thr Trp Tyr Asn Gln Leu Gly Ser Thr Phe Ile Val Thr
            20                  25                  30

Ala Gly Ala Asp Gly Ala Leu Thr Gly Thr Tyr Glu Ser Ala Val Gly
        35                  40                  45

Asn Ala Glu Ser Arg Tyr Val Leu Thr Gly Arg Tyr Asp Ser Ala Pro
    50                  55                  60

Ala Thr Asp Gly Ser Gly Thr Ala Leu Gly Trp Thr Val Ala Trp Lys
65                  70                  75                  80

Asn Asn Tyr Arg Asn Ala His Ser Ala Thr Thr Trp Ser Gly Gln Tyr
                85                  90                  95

Val Gly Gly Ala Glu Ala Arg Ile Asn Thr Gln Trp Leu Leu Thr Lys
            100                 105                 110

Gly Thr Thr Glu Ala Asn Ala Trp Lys Ser Thr Leu Val Gly His Asp
        115                 120                 125

Thr Phe Thr Lys Val Lys Pro Ser Ala Ala Ser Ile Asp Ala Ala Lys
    130                 135                 140

Lys Ala Gly Val Asn Asn Gly Asn Pro Leu Asp Ala Val Gln Gln
145                 150                 155